# DEMANDE DE BREVET EUROPEEN

(11) **EP 1 428 504 A1**
(43) Date de publication de la demande: **16.06.2004**
(21) Numéro de dépôt: 03292898.8
(22) Date de dépôt: 21.11.2003
(51) Int. Cl.: A61K 7/13

(54) **Composition tinctorial contenant au moins un composé hétéroaromatique dialdehyde et au moins un composé azoté**

(30) Priorité: 29.11.2002 FR 0215058
(71) Demandeur: L'OREAL, 75008 Paris (FR)
(72) Inventeur: Plos, Grégory, 75011 Paris (FR)
(74) Mandataire: Wattremez, Catherine

(57) **Abrégé**

L'invention a pour objet une composition tinctoriale pour la teinture des fibres kératiniques comprenant au moins un composé hétérocyclique contenant au moins deux fonctions aldéhyde en position α ou β ou γ et au moins un composé azoté. L'invention a aussi pour objet l'utilisation de cette composition pour la teinture des fibres kératiniques ainsi que le procédé de teinture mettant en oeuvre cette composition.

## Description

La présente invention a pour objet une composition tinctoriale pour la teinture des fibres kératiniques, en particulier des fibres kératiniques humaines telles que les cheveux, comprenant au moins un composé hétérocyclique contenant au moins deux fonctions aldéhyde en position α ou β ou γ et au moins un composé azoté.

Dans le domaine de la teinture des fibres kératiniques, en particulier des fibres kératiniques humaines telles que les cheveux, il existe deux modes de coloration qui présentent chacun leurs avantages et leurs inconvénients :
- La *coloration directe* ou *coloration semi-permanente* consiste à amener la couleur par une molécule colorée qui s'adsorbe à la surface des fibres kératiniques et/ou pénètre par diffusion dans les couches superficielles de celles-ci. Les temps de pose sont généralement assez courts et les conditions douces de teinture préservent l'intégrité des fibres kératiniques, mais les colorations obtenues par ce mode de teinture résistent mal au lavage et s'estompent après seulement 4 ou 5 shampooings. De plus, les gammes des nuances obtenues sont en général réduites.
- La *coloration d'oxydation* ou *coloration permanente* met en oeuvre la condensation oxydative de molécules incolores ou faiblement colorées, appelées bases d'oxydation, telles que des ortho ou para-phénylènediamines, des ortho ou para-aminophénols, des composés hétérocycliques en présence d'un agent oxydant. Cette réaction conduit à la formation de composés colorés polymères dans les fibres kératiniques. Le principal avantage de la teinture d'oxydation réside dans la longévité des colorations obtenues, en particulier dans la résistance aux lavages et aux agents extérieurs tels que la lumière, les intempéries, les ondulations permanentes, la transpiration et les frottements, ainsi que dans l'obtention d'une large palette de nuances. Cependant, les conditions chimiques de teinture, telles que le pH et un milieu oxydant, entraînent une dégradation des fibres kératiniques. Par ailleurs, ce mode de teinture nécessite des temps de pose relativement longs.

Il existe déjà des systèmes de coloration des fibres kératiniques mettant en oeuvre un composé aldéhyde en présence d'une amine sans utiliser d'agents oxydants qui dégradent les cheveux. Il a été proposé dans le brevet FR 2 787 705 des compositions de teinture des fibres kératiniques comprenant une amine cationique aliphatique et un composé choisi parmi un aldéhyde, une cétone, une quinone et un dérivé de la di-imino-isoindoline ou de la 3-amino-isoindolone et dans le brevet FR 2 787 706 des compositions de teinture des fibres kératiniques comprenant une amine cationique hétérocyclique et un composé choisi parmi un aldéhyde, une cétone, une quinone et un dérivé de la di-imino-isoindoline ou de la 3-amino-isoindolone.

Les brevets US 3 871 818 et US 3 904 357 décrivent un procédé de teinture des cheveux par des dialdéhydes en présence d'au moins un composé azoté. Ces documents divulguent en particulier l'association, d'une part d'un xanthènedicarboxaldéhyde, et d'autre part d'un pipéridinedicarboxaldéhyde, avec un composé contenant un atome d'azote.

Cependant, les colorations obtenues en mettant en oeuvre ces compositions tinctoriales ne sont pas toujours assez puissantes, esthétiques, chromatiques ou suffisamment résistantes aux différentes agressions que peuvent subir les cheveux.

Le but de la présente invention est de fournir de nouvelles compositions tinctoriales pour la teinture de fibres kératiniques ne présentant pas les inconvénients de celles de l'art antérieur. En particulier, le but de la présente invention est de fournir un nouveau système de coloration qui présente à la fois les avantages de la ténacité, en particulier aux lavages répétés et du respect de la fibre capillaire.

Ce but est atteint avec la présente invention qui a pour objet une composition tinctoriale des fibres kératiniques comprenant, dans un milieu de teinture approprié :
- au moins un composé hétérocyclique polyaldéhyde de formule (I) comprenant au moins deux fonctions aldéhyde situées en position α, β ou γ ou l'un de ses sels d'addition correspondants : 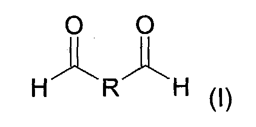
où R représente un groupement mono ou polyhétérocyclique divalent insaturé, condensé ou non condensé, aromatique ou non aromatique, comportant de 5 à 30 chaînons et contenant un ou plusieurs hétéroatomes choisis parmi l'azote, le soufre, l'oxygène et/ou le phosphore,
ces groupements hétérocycliques pouvant être substitués par des groupements halogéno, alkyle en C₁-C₄, hydroxy, alcoxy en C₁-C₄, alkylthio en C₁-C₄, amino, mono ou di(C₁-C₄)alkylamino, alkylcarbonyle en C₁-C₄, hydrogénocarbonyle, alcoxycarbonyle en C₁-C₄, nitro, sulfonato, ammonio, tri(C₁-C₄)alkylammonio, imidazolio, pyridinio, benzothiazolio;
- au moins un composé azoté de formule (II) ou l'un de ses sels d'addition correspondants :

   NR₁R₂R₃ (II)

   où R₁, R₂ et R₃ indépendamment l'un de l'autre sont choisis parmi :
   - un atome d'hydrogène ;
   - un groupement hydroxy ;
   - un groupement alcoxy en C₁-C₄ ;
   - une chaîne aliphatique en C₁₋₃₀, ramifiée ou non, pouvant présenter des doubles ou triples liaisons, éventuellement substituée par un ou plusieurs groupements hydroxy, amino, ou halogéno ;
   - un groupement mono ou polyaromatique, condensé ou non condensé, comprenant de 6 à 50 atomes de carbone ;
   - un groupement mono ou polyhétérocyclique, condensé ou non condensé, aromatique ou non aromatique, comportant de 5 à 30 chaînons et contenant un ou plusieurs hétéroatomes choisis parmi l'azote, le soufre, l'oxygène et/ou le phosphore ; ces groupements hétérocycliques pouvant être substitués par des groupements halogéno, alkyle en C₁-C₄, hydroxy, alcoxy en C₁-C₄, alkylthio en C₁-C₄, amino, mono ou di(C₁-C₄)alkylamino, alkylcarbonyl en C₁-C₄, hydrogénocarbonyle, carboxy, nitro, sulfonato.

Les compositions selon l'invention permettent d'obtenir des nuances tenaces, en particulier aux shampooings. Ces compositions présentent également l'avantage de limiter la dégradation des fibres capillaires car la fibre kératinique n'est pas en contact avec des agents oxydants puissants tels que l'eau oxygénée utilisée habituellement pour la teinture d'oxydation.

L'invention a aussi pour objet un procédé de teinture des fibres kératiniques, en particulier des fibres kératiniques humaines telles que les cheveux, utilisant cette composition, ainsi qu'un dispositif de teinture permettant de mettre en oeuvre ce procédé.

Un autre objet de l'invention est l'utilisation de la composition de la présente invention pour la teinture des fibres kératiniques, en particulier des fibres kératiniques humaines telles que les cheveux.

Par position α, β ou γ, on entend au sens de la présente invention des substitutions respectivement sur deux atomes voisins, sur deux atomes séparés par un atome ou par deux autres atomes, les atomes en question étant soit des atomes de carbone soit des hétéroatomes.

Dans le cadre de la présente invention, le terme "condensé" signifie au moins deux cycles accolés présentant au moins deux atomes communs.

On entend par radical alkyle (alk) un radical alkyle linéaire ou ramifié par exemple méthyle, éthyle, n-propyle, iso-propyle, butyle. Un radical alcoxy est un radical alk-O-, un radical alkylcarbonyl est un radical alk-CO-, un radical alcoxycarbonyle est un radical alk-O-CO-, un radical alkylthio est un radical alk-S-, un radical mono ou dialkylamino est un radical (alk)ₙN- avec n = 1 ou 2 dans chacune de ces définitions le radical alkyle ayant la définition donnée ci-dessus.

Un radical sulfonato est un radical -SO₃⁻. Un radical trialkylammonio est un radical (alk)₃N⁺- avec le radical alkyle ayant la définition donnée ci-dessus. Les radicaux imidazolio, pyridinio et benzothiazolio sont les radicaux cationiques correspondant aux cations imidazolium, pyridinium et benzothiazolium.

Un groupement halogéno désigne un atome d'halogène choisi de préférence parmi le chlore, le brome et l'iode.

Un groupement mono ou polyaromatique, condensé ou non condensé, comprenant de 6 à 50 atomes de carbone peut être par exemple un cycle benzénique, naphtalénique, anthracénique. Un groupement mono ou polyhétérocyclique, condensé ou non condensé, aromatique ou non aromatique, comportant de 5 à 30 chaînons et contenant un ou plusieurs hétéroatomes peut être par exemple un cycle thiophène, benzofurane, benzothiophène, indole, bispyridine, benzopyrane, quinolines, pyrazole, pyridine, pyrrole, furane, imidazole, benzimidazole. Le groupement polyhétérocyclique peut être condensé ou substitué par un ou plusieurs carbocycles.

Selon un mode de réalisation particulier de la composition de l'invention, le composé hétérocyclique polyaldéhyde de formule (I) est de préférence un dialdéhyde.

Selon un autre mode de réalisation particulier de la composition de l'invention, le composé hétérocyclique polyaldéhyde de formule (I) est de préférence non condensé.

Selon un autre mode de réalisation particulier de la composition de l'invention, le composé hétérocyclique polyaldéhyde de formule (I) est choisi parmi les thiophènedicarboxaldéhydes et leurs dérivés, les pyridinedicarboxaldéhydes et leurs dérivés, les pyrroledicarboxaldéhydes et leurs dérivés et les furannedicarboxaldéhydes et leurs dérivés.

A titre d'exemple, le composé hétérocyclique polyaldéhyde de formule (I) est choisi parmi le 2,3-thiophènedicarboxaldéhyde, le 2,6-pyridinedicarboxaldéhyde, le 3,4-diméthyl-2,5-pyrroledicarboxaldéhyde, le 2,5-thiophènedicarboxaldéhyde et le 2,5-diformyl-3,4-furannedicarboxaldéhyde.

De préférence, le composé hétérocyclique polyaldéhyde de formule (I) est un composé hétérocyclique à 5 chaînons, par exemple le 2,3-thiophènedicarboxaldéhyde, le 3,4-diméthyl-2,5-pyrroledicarboxaldéhyde et le 2,5-thiophènedicarboxaldéhyde.

Selon un mode de réalisation particulier de la composition de l'invention, lorsque l'un au moins des radicaux R₁, R₂ ou R₃ de la formule (II) représente un groupement mono ou polyaromatique, condensé ou non condensé, comprenant de 6 à 50 atomes de carbone, ledit groupement aromatique est non substitué ou substitué par un ou plusieurs groupements halogéno, alkyle en C₁-C₄, hydroxy, alcoxy en C₁-C₄, alkylthio en C₁-C₄, amino, mono ou di(C₁-C₄)alkylamino, alkylcarbonyl en C₁-C₄, hydrogénocarbonyle, carboxy, nitro, sulfonato.

Selon un mode de réalisation particulier de la composition de l'invention, le composé azoté de formule (II) est choisi parmi les monoalcanolamines, les dialcanolamines, les trialcanolamines, les alkylalcanolamines, les dialkylalcanolamines, les alkyldialcanolamines.

De préférence, le composé azoté de formule (II) est choisi parmi les monoalcanolamines en C₁-C₃, les di(C₁-C₃)alcanolamines, les tri(C₁-C₃)alcanolamines, les (alkyl en C₁-C₄)(alcanol en C₁-C₃)amines, les (di(C₁-C₄)alkyl)(alcanol en C₁-C₃)amines, les (alkyl en C₁-C₄)(di(C₁-C₃)alcanol)amines.

Selon ce dernier mode de réalisation, parmi les composés de formule (II) utilisables selon l'invention, on peut citer la monoéthanolamine, la triéthanolamine, le 2-méthylpropanol-1 et la monoisopropanolamine.

Selon un autre mode de réalisation particulier de la composition de l'invention, le composé de formule (II) est choisi parmi les amines et les diamines aromatiques.

A titre d'exemple, on peut citer les para-phénylènediamines, les bis-phénylalkylènediamines, les para-aminophénols, les ortho-aminophénols, les méta-phénylènediamines, les méta-aminophénols, les bases et les coupleurs hétérocycliques contenant au moins une fonction amine.

Parmi les para-phénylènediamines utilisables selon l'invention, on peut citer la para-phénylènèdiamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl paraphénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide sont particulièrement préférées.

Selon un mode de réalisation préféré, le composé azoté de formule (II) est l'ammoniaque.

Les concentrations du composé hétérocyclique polyaldéhyde de formule (I) et du composé azoté de formule (II) dans la composition varient largement en fonction de la nuance désirée. Cependant, le composé hétérocyclique polyaldéhyde de formule (I) est en général présent en quantité comprise entre 0,01 et 30 % en poids, rapporté au poids total de la composition tinctoriale, de préférence entre 0,05 et 20 % et la concentration en composé azoté de formule (II) est généralement comprise entre 0,01 et 30 % en poids, rapporté au poids total de la composition tinctoriale, de préférence entre 0,05 et 20%.

La composition tinctoriale conforme à l'invention peut en outre contenir un ou plusieurs colorants directs pouvant notamment être choisis parmi les colorants nitrés de la série benzénique, les colorants directs azoïques, les colorants directs méthiniques. Ces colorants directs peuvent être de nature non ionique, anionique ou cationique.

La composition de la présente invention peut aussi comprendre une ou plusieurs bases d'oxydation classiquement utilisées en teinture d'oxydation ainsi que des coupleurs autres que ceux pouvant jouer le rôle de composé azoté de formule (II). A titre d'exemple, ces bases d'oxydation sont choisies parmi les para-diphénols et les bases hétérocycliques ne contenant pas de fonction amine. Les coupleurs sont choisis parmi les méta-diphénols et les coupleurs hétérocycliques ne contenant pas de fonction amine.

Le milieu approprié pour la teinture, appelé aussi support de teinture, est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci-dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture conforme à l'invention ne soient pas, ou substantiellement pas, altérées par la ou les adjonctions envisagées.

Le pH de la composition tinctoriale conforme à l'invention est généralement compris entre 4 et 11, et de préférence entre 5 et 10. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Le ou les composés azotés de formule (II) jouent le rôle d'agents alcalinisants. Le pH peut cependant être ajusté par l'ajout d'autres agents alcalinisants tels que les carbonates alcalins et les hydroxydes de sodium ou de potassium.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

Le procédé de teinture des fibres kératiniques de la présente invention consiste à appliquer une composition (a) comprenant, dans un milieu cosmétique approprié à la teinture des cheveux, au moins un composé hétérocyclique polyaldéhyde de formule (I) et une composition (b) comprenant, dans un milieu cosmétique approprié à la teinture des cheveux, au moins un composé azoté de formule (II) sur les fibres kératiniques pendant un temps de pose suffisant pour obtenir la coloration désirée.

Selon un mode de réalisation particulier, les compositions (a) et (b) sont mélangées juste avant emploi et le mélange ainsi obtenu est appliqué sur les fibres kératiniques pendant un temps de pose suffisant pour obtenir la coloration désirée.

Selon une variante, le procédé de teinture des fibres kératiniques consiste à appliquer les compositions (a) et (b) successivement sur les fibres kératiniques pendant un temps de pose suffisant pour obtenir la coloration désirée. L'ordre d'application de ces compositions est indifférent.

Selon un mode de réalisation particulier, les fibres kératiniques sont rincées entre l'application des deux compositions.

Le temps de pose de chacune des compositions (a) ou (b) ou de leur mélange est généralement fixé entre 5 minutes et 1 heure, de préférence entre 5 minutes et 30 minutes.

La température d'application des différentes compositions est généralement fixée entre la température ambiante et 80 °C, de préférence entre la température ambiante et 60 °C.

Le dispositif à plusieurs compartiments de l'invention comprend un premier compartiment qui contient une composition comprenant au moins un composé hétérocyclique dialdéhyde de formule (I) et un deuxième compartiment qui contient une composition comprenant au moins un composé azoté de formule (II).

Ce dispositif peut être équipé d'un moyen permettant de délivrer sur les cheveux le mélange souhaité, tel que les dispositifs décrits dans le brevet FR-2 586 913 au nom de la demanderesse. A partir de ce dispositif, il est possible de teindre les fibres kératiniques à partir d'un procédé tel que décrit ci-dessus.

La présente invention a aussi pour objet l'utilisation d'une composition comprenant au moins un composé hétérocyclique polyaldéhyde de formule (I) et au moins un composé azoté de formule (II) pour la teinture des fibres kératiniques.

Les exemples qui suivent servent à illustrer l'invention sans toutefois présenter un caractère limitatif.

### EXEMPLES

### Exemple 1

Une composition tinctoriale est réalisée comme indiqué ci-dessous :

| Composés | Composition 1 |
|---|---|
| 2,3-thiophène dicarboxaldéhyde | 6.10⁻³ mole |
| Ammoniaque | 0,8 g |
| Eau distillée | q.s.p. 100 g |

Cette composition est appliquée sur des mèches de cheveux gris à 90% de cheveux blancs naturels ou permanentés. Après un temps de pose de 30 minutes à 50 °C, les mèches sont shampouinées, rincées puis séchées.
La coloration obtenue est mesurée au moyen d'un spectrocolorimètre CM2002 Minolta (CSE, illuminant D65, angle 10°).
Les résultats colorimétriques sont donnés ci-dessous :

| | L* | a* | b* | Couleur |
|---|---|---|---|---|
| Mèche colorée naturelle | 30,50 | 0,85 | 7,75 | marron |
| Mèche colorée permanentée | 28,25 | 7,10 | 2,85 | marron |

### Exemple 2

Les compositions 2 et 3 suivantes sont réalisées :

| Composés | Composition 2 | Composition 3 |
|---|---|---|
| 3, 4-diméthyl 2, 5-pyrroledicarboxaldéhyde | 3.10⁻³ mole | - |
| 2, 5 thiophène dicarboxaldéhyde | - | 3.10⁻³ mole |
| Paraphénylènediamine | 9.10⁻³ mole | 9.10⁻³ mole |
| NaOH | q.s.p. pH 9 | q.s.p. pH 9 |
| Eau distillée | q.s.p. 100 g | q.s.p. 100 g |

Ces compositions sont appliquées sur des mèches de cheveux gris à 90 % de cheveux blancs naturels. Après un temps de pose de 30 minutes à température ambiante, les mèches sont shampouinées, rincées puis séchées.
La couleur des mèches est mesurée au moyen d'un spectrocolorimètre CM2002 Minolta (CSE, illuminant D65, angle 10°).
Les résultats colorimétriques sont donnés ci-dessous :

| Mèches | L* | a* | b* | Couleur |
|---|---|---|---|---|
| Composition 2 | 23,60 | 14,05 | 9,45 | cuivré |
| Composition 3 | 26,15 | 12,05 | 12,90 | cuivré |

## Revendications

1. Composition tinctoriale des fibres kératiniques comprenant, dans un milieu de teinture approprié :
- au moins un composé hétérocyclique polyaldéhyde de formule (I) comprenant au moins deux fonctions aldéhyde situées en position α, β ou γ ou l'un de ses sels d'addition correspondants : 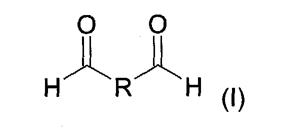 où R représente un groupement mono ou polyhétérocyclique divalent insaturé, condensé ou non condensé, aromatique ou non aromatique, comportant de 5 à 30 chaînons et contenant un ou plusieurs hétéroatomes choisis parmi l'azote, le soufre, l'oxygène et/ou le phosphore,
ces groupements hétérocycliques pouvant être substitués par des groupements halogéno, alkyle en C₁-C₄, hydroxy, alcoxy en C₁-C₄, alkylthio en C₁-C₄, amino, mono ou di(C₁-C₄)alkylamino, alkylcarbonyle en C₁-C₄, hydrogénocarbonyle, alcoxycarbonyle en C₁-C₄, nitro, sulfonato, ammonio, tri(C₁-C₄)alkylammonio, imidazolio, pyridinio, benzothiazolio;
- au moins un composé azoté de formule (II) ou l'un de ses sels d'addition correspondants :
NR₁R₂R₃ (II)
où R₁, R₂ et R₃ indépendamment l'un de l'autre sont choisis parmi :
- un atome d'hydrogène ;
- un groupement hydroxy ;
- un groupement alcoxy en C₁-C₄ ;
- une chaîne aliphatique en C₁₋₃₀, ramifiée ou non, pouvant présenter des doubles ou triples liaisons, éventuellement substituée par un ou plusieurs groupements hydroxy, amino ou halogéno ;
- un groupement mono ou polyaromatique, condensé ou non condensé, comprenant de 6 à 50 atomes de carbone ;
- un groupement mono ou polyhétérocyclique, condensé ou non condensé, aromatique ou non aromatique, comportant de 5 à 30 chaînons et contenant un ou plusieurs hétéroatomes choisis parmi l'azote, le soufre, l'oxygène et/ou le phosphore ;
ces groupements hétérocycliques pouvant être substitués par des groupements halogéno, alkyle en C₁-C₄, hydroxy, alcoxy en C₁-C₄, alkylthio en C₁-C₄, amino, mono ou di(C₁-C₄)alkylamino, alkylcarbonyl en C₁-C₄, hydrogénocarbonyle, carboxy, nitro, sulfonato.

2. Composition selon la revendication 1, dans laquelle le composé hétérocyclique polyaldéhyde de formule (I) est un dialdéhyde.

3. Composition selon la revendication 1 ou 2, dans laquelle le composé hétérocyclique polyaldéhyde de formule (I) est non condensé.

4. Composition selon la revendication 3, dans laquelle le composé hétérocyclique polyaldéhyde de formule (I) est choisi parmi les thiophènedicarboxaldéhydes et leurs dérivés, les pyridinedicarboxaldéhydes et leurs dérivés, les pyrroledicarboxaldéhydes et leurs dérivés et les furanedicarboxaldéhydes et leurs dérivés.

5. Composition selon la revendication 4, dans laquelle le composé hétérocyclique polyaldéhyde de formule (I) est choisi parmi le 2,3-thiophènedicarboxaldéhyde, le 2,6-pyridinedicarboxaldéhyde, le 3,4-diméthyl-2,5-pyrroledicarboxaldéhyde, le 2,5-thiophènedicarboxaldéhyde, le 2,5-diformyl-3,4-furannedicarboxaldéhyde.

6. Composition selon la revendication 3, dans laquelle le composé hétérocyclique polyaldéhyde de formule (I) est un composé hétérocyclique à 5 chaînons.

7. Composition selon la revendication 6, dans laquelle le composé hétérocyclique polyaldéhyde de formule (I) est choisi parmi le 2,3-thiophènedicarboxaldéhyde, le 3,4-diméthyl-2,5-pyrroledicarboxaldéhyde et le 2,5-thiophènedicarboxaldéhyde.

8. Composition selon l'une quelconque des revendications précédentes, dans laquelle lorsque l'un au moins des radicaux R₁, R₂ ou R₃ de la formule (II) représente un groupement mono ou polyaromatique, condensé ou non condensé, comprenant de 6 à 50 atomes de carbone, ledit groupement aromatique est non substitué ou substitué par un ou plusieurs groupements halogéno, alkyle en C₁-C₄, hydroxy, alcoxy en C₁-C₄, alkylthio en C₁-C₄, amino, mono ou di(C₁-C₄)alkylamino, alkylcarbonyl en C₁-C₄, hydrogénocarbonyle, carboxy, nitro, sulfonato.

9. Composition selon l'une quelconque des revendications précédentes, dans laquelle le composé azoté de formule (II) est choisi parmi les monoalcanolamines, les dialcanolamines, les trialcanolamines, les alkylalcanolamines, les dialkylalcanolamines, les alkyldialcanolamines, les amines et diamines aromatiques, l'ammoniaque.

10. Composition selon la revendication 9, dans laquelle le composé azoté de formule (II) est choisi parmi les monoalcanolamines en C₁-C₃, les di(C₁-C₃)alcanolamines, les tri(C₁-C₃)alcanolamines, les (alkyl en C₁-C₄)(alcanol en C₁-C₃)amines , les (di(C₁-C₄)alkyl)(alcanol en C₁-C₃)amines , les (alkyl en C₁-C₄)(di(C₁-C₃)alcanol)amines .

11. Composition selon la revendication 10, dans laquelle le composé azoté de formule (II) est choisi parmi la monoéthanolamine, la triéthanolamine, le2-méthylpropanol-1 et la monoisopropanolamine,

12. Composition selon la revendication 9, dans laquelle le composé azoté de formule (II) est choisi parmi les amines et les diamines aromatiques.

13. Composition selon la revendication 12, dans laquelle le composé azoté de formule (II) est un dérivé de la para-phénylènediamine.

14. Composition selon la revendication 13, dans laquelle le composé azoté de formule (II) est choisi parmi la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

15. Composition selon la revendication 14, dans laquelle le composé azoté de formule (II) est choisi parmi la para-phénylènediamine, la para-toluènediamine, la 2-isopropyl para-phénytènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétylaminoéthyloxy para-phénylènediamine, et leurs sels d'addition avec un acide.

16. Composition selon la revendication 9, dans laquelle le composé azoté de formule (II) est l'ammoniaque.

17. Composition selon l'une quelconque des revendications précédentes, dans laquelle la concentration en composé hétérocyclique polyaldéhyde de formule (I) est comprise entre 0,01 et 30 % en poids, rapporté au poids total de la composition.

18. Composition selon l'une quelconque des revendications précédentes, dans laquelle la concentration en composé azoté de formule (II) est comprise entre 0,01 et 30 % en poids, rapporté au poids total de la composition.

19. Composition selon l'une quelconque des revendications précédentes, dans laquelle le pH est compris entre 4 et 11.

20. Procédé de teinture des fibres kératiniques, **caractérisé en ce qu'**une composition (a) comprenant, dans un milieu cosmétique approprié à la teinture des cheveux, au moins un composé hétérocyclique polyaldéhyde de formule (I) tel que défini dans l'une quelconque des revendications 1 à 7 et une composition (b) comprenant, dans un milieu cosmétique approprié à la teinture des cheveux, au moins un composé azoté de formule (II) tel que défini dans l'une quelconque des revendications 1 et 8 à 16 sont appliquées séparément ou après mélange sur les fibres kératiniques pendant un temps de pose suffisant pour obtenir la coloration désirée.

21. Procédé selon la revendication 20, dans lequel les compositions (a) et (b) sont mélangées juste avant emploi et le mélange ainsi obtenu est appliqué sur les fibres kératiniques pendant un temps de pose suffisant pour obtenir la coloration désirée.

22. Procédé selon la revendication 20, dans lequel les compositions (a) et (b) sont appliquées successivement sur les fibres kératiniques pendant un temps de pose suffisant pour obtenir la coloration désirée, l'ordre d'application des compositions (a) et (b) étant indifférent.

23. Procédé selon la revendication 22, dans lequel les fibres kératiniques sont rincées entre l'application de la composition (a) et l'application de la composition (b).

24. Dispositif à plusieurs compartiments, dans lequel un premier compartiment contient une composition comprenant au moins un composé hétérocyclique dialdéhyde de formule (I) tel que défini dans l'une quelconque des revendications 1 à 7 et un deuxième compartiment contient une composition comprenant au moins un composé azoté de formule (II) tel que défini dans l'une quelconque des revendications 1 et 8 à 16.

25. Utilisation d'une composition telle que définie selon l'une quelconque des revendications 1 à 19 pour la teinture des fibres kératiniques.
